# EUROPEAN PATENT SPECIFICATION

(11) **EP 1 265 598 B1**
(45) Date of publication and mention of the grant of the patent: **02.08.2006**
(21) Application number: 01914018.5
(22) Date of filing: 22.03.2001
(51) Int. Cl.: A61K 9/16, A61K 39/00

(54) **PHARMACEUTICAL COMPOSITION FOR APPLICATION TO MUCOSAL SURFACES**
ARZNEIMITTEL ZUR VERABREICHUNG AN SCHLEIMHÄUTE
COMPOSITION PHARMACEUTIQUE POUR ADMINISTRATION SUR DES SURFACES MUQUEUSES

(30) Priority: 22.03.2000 GB 0006770; 16.01.2001 GB 0101094
(43) Date of publication of application: 18.12.2002
(73) Proprietor: THE SECRETARY OF STATE FOR DEFENCE, Salisbury SP4 0JQ (GB)
(72) Inventor: ALPAR, Hazire Oya, Aston Triangle Birmingham B4 7ET (GB); EYLES, James Edward, Salisbury Wiltshire SP4 0JQ (GB); WILLIAMSON, Ethel Diane, Salisbury Wiltshire SP4 0JQ (GB)
(74) Representative: Greaves, Carol Pauline
(86) International application number: PCT/GB2001/001248
(87) International publication number: WO 2001/070200

(56) References cited:
- WO-A-94/15636
- J.E. EYLES ET AL.: "Generation of protective immune responses to plague by mucosal administration of microsphere coencapsulated recombinant subunits" JOURNAL OF CONTROLLED RELEASE, vol. 63, no. 1-2, January 2000 (2000-01), pages 191-200, XP004185083 Amsterdam (NL)
- A. K. HILBERT ET AL.: "Biodegradable microspheres containing influenza A vaccine: immune response in mice" VACCINE, vol. 17, no. 9-10, 5 March 1999 (1999-03-05), pages 1065-1073, XP004158227 Guildford (GB)

## Description

The present invention relates to a composition which is particularly useful for delivering pharmaceuticals such as vaccines to mucosal surfaces, for example intranasal formulations. The invention further comprises the use of the composition according to claim 1 in the preparation of a vaccine for the production of a protective immune response in a mammal and methods of preparing the composition.

A prime objective in the field of vaccination is the development of a non-parenteral immunisation regimen which facilitate induction of comparable levels of systemic immunity to that elicited by conventional sub-cutaneous and intramuscular injections.

The nasopharyngeal passages and pulmonary regions of the respiratory tract represent potential targets for the systemic delivery of peptidergic drugs and vaccines. The relative ease with which therapeutic agents can be inhaled, or introduced into the nose, make these modes of immunisation attractive in terms of probable patient compliance. Furthermore, respiratory mucosae offer certain morphological, physiological and immunological advantages over other non-parenteral sites in terms of immunisation, particularly against pathogenic entitities which affect or utilise mucosal surfaces as portals of entry. This is because effective vaccination against these pathogens normally requires mucosae to the adequately protected with locally produced antibodies of the secretory IgA (sIgA) isotype. Whilst mucosal surfaces are usually poorly protected with IgA following parenteral administration of vaccines, it is now apparent that successful delivery of antigenic material to immunoresponsive elements in mucosa-associated lymphoid tissue (MALT) can result in vigorous stimulation of the mucosal arm of the immune system. By means of the common mucosal immune system (CMIS) it is feasible that several anotomically disparate mucosal surfaces could be protected through mucosal administration of a vaccine at a single site. Mucosal vaccination offers the added advantage that some degree of systemic immunity can be induced in concert with local responses due to translocation of antigenic material from sub-epithelial compartments to systemic immunoresponsive tissues such as the spleen.

Despite the logistical and immunological factors which favour non-parenteral immunisation, simple mucosal application of antigenic proteins, for example in the gastrointestinal or respiratory tracts, is usually ineffectual in terms of vaccination. Enzymatic or chemical destruction, combined with poor absorption into sub-epithelial compartments dictate that mucosally administered vaccines usually require some form of adjuvant or delivery vehicle. One approach is to encapsulate antigenic material within microparticulate polymeric carriers, such as poly-DL-lactide (PLA) microspheres (Vaccine 1994, 12, 5-11). Such procedures serve to protect labile vaccines from lumenal degradation and enhance adsorption into mucosal and systemic compartments (J.H. Eldridge et al., Seminars in Hematology, (1993), 30, 16-25). There is good evidence that microencapsulation may also adjuvantise by converting soluble antigenic molecules into particulate species, thus promoting vaccine uptake into antigen presenting cells (APC)(Y. Tabata et al., Adv. Polym. Sci. (1990), 94, 107-141, L. Vidard et al., J. Immunol. (1996), 156, 2809-2818, N. Van Rooijen, Immunol. Today (1990) 11, 436-439) or microfold cells (M-cells) in lymphoid follicles (R.I. Walker et al., Vaccine, 12, 387, 1994, D.T. O'Hagan et al,, Vaccine, 1989, 7, 421-424, P.G. Jenkins et al., J. Drug Targetting, 1995, 3, 79-81).

J.E. Eyles et al., J. Contr. Release, 63 (2000) 101-200, discloses a pharmaceutical composition (vaccine) for administration to mucosal surfaces (intranasal administration) comprising recombinant subunit antigens F1 and V from *Yersinia pestis* which are coencapsulated in biodegradable poly(L-lactide) (PLLA) microspheres.

Although comparatively under-investigated; the intra-nasal (i.n.) route is an attractive one for the mucosal delivery of vaccinal entities. The nasal epithelium is accessible and is less exclusive to high molecular weight molecules.

The thickness of the mucus blanket covering respiratory epithelium is relatively thin compared to that of other mucosae, for example the gut where it is in the region of 500 times thicker. Substantially reduced concentrations of proteolytic enzymes and extremes of pH exist in the respiratory tract compared with the gastrointestinal tract.

Furthermore, it is now delineated that nasal associated lymphoids tissues (NALT) have a lymphoepithelium which, like that in the intestinal mucosa, contain M-cells for selective antigen uptake (P. Brandenburg, Immunology of the Lung and Upper Respiratory Tract, (ed. Bienenstock J.) McGraw-Hill, New York, 1984, 28-95). Hence NALT plays an analogous role to other MALT, such as the gut associated lymphoid tissues (GALT), in terms of antigen surveillance and induction of mucosal and systemic immunological responses.

The use of high molecular weight polymers in the encapsulation of a tetanus vaccine for intramuscular administration has been described (Vaccine 1994, 12, 4, 299-306). A formulation of microencapsulated ricin toxoid vaccine which is applied intranasally has also been described (Vaccine 1994, 14, 11 1031). However, in that case, high molecular weight polymer microspheres (94KDa) were less effective than those prepared from a copolymer of lower molecular weight (72KDa).

The use of high molecular weight polymers (PLA100KDa) in the production of microspheres for use in generating immune responses against *Yersinia pestis* has been described previously in Immunology 1997, 92, Suppl. 1, 56 and J. Pharm. Pharmacol. 1997, 49, Suppl 4, 85. In those cases however, multiple doses of the compositions were administered (i.e. and initial dose followed by either one or two booster doses) intranasally.

It is desirable to minimise or eliminate the need for booster doses of pharmaceuticals such as vaccines. This is because it is inconvenient and costly to see patients on a repeated basis.

Attempts have also been made to enhance the efficacy of pharmaceutical compositions such as vaccine compositions by use of adjuvants. US Patent No. 5,643,605 discloses methods and compositions in which principally adjuvants are encapsulated in low viscosity polymers to form microspheres having a median diameter of from about 20 to 100µm. Such compositions will therefore include microspheres of greater than 20µm diameter.

The applicants have found that a particular microencapsulated formulation produces high levels of efficacy when administered by way of a mucosal surface. For example, in the case of vaccines, these may generally be administered in a single dose, without the need for adjuvants.

By "single dose" is meant that the pharmaceutical and in particular, a vaccine is applied without boosting.

Thus the invention provides a pharmaceutical composition for administration to mucosal surfaces, which composition comprises an agent which is capable of generating a biological effect, a first amount of said agent being encapsulated within microspheres which comprise a polymer which has a molecular weight in excess of 94kDa and a maximum diameter of 20µm, and a second amount of said agent being in a form which has a higher bioavailability than said first amount.

The biological effect is suitably one which generates a protective immune response such as a vaccine but other pharmaceutically active compounds may be employed. In particular, other suitable biologically active compounds are pharmaceuticals such as CNS active drugs, analgesics such as centrally acting analgesics, pain relievers such as morphine analogues, and hormones such as insulin.

The higher bioavailability of the second amount of the active agent means that when it is administered, it is very swiftly incorporated into the system of the recipient. It therefore aids in achieving a fast and high immune response, which is then reinforced by the slower release of material from the microspheres. The high molecular weight polymeric microspheres tend to delay release of the agent encapsulated therewith.

Suitably the said second amount of said agent is free in the composition or is at least partially adsorbed onto, or weakly associated with the surface of said microspheres.

Alternatively, it may encapsulated in a readily dispersible microsphere or vesicle which either surrounds or is separate from the high molecular weight polymeric microspheres which form part of the composition of the invention.

Suitably, the polymer has a molecular weight of 100KDa or more. Suitably the polymer comprises a "structural polymer" which is water insoluble polymer, capable of forming structures such as microspheres. Such polymers may be consisted to be matrix materials and are soluble only in organic solvents. A particularly suitable polymer for use in the compositions of the invention comprises poly-(L-lactide) or PLA but other high molecular weight polymeric material such as poly(lactic/glycolic acid) PGLA may be employed. Structural polymers and in particular, PLA, of high crystallinity, for example which is more than 70% crystalline, are preferred. Such polymers are of high viscosity, for example with a viscosity of greater than 1.2dL/g, and more preferably in greater than 1.5dL/g.

However preferably they comprise at least 70%, more preferably at least 80% and most preferably at least 95% of the high molecular weight polymers as described above. In particular, the polymer used is 100% high molecular weight PLA.

The microspheres may optionally further comprise agents which stabilise emulsions such as polyvinylalcohol or methyl cellulose, and preferably polyvinylalcohol.

Suitably the microspheres do not include low molecular weight structural polymers such low molecular weight PLA or PGLA as mentioned above.

Microspheres of this type are quite small, having a maximum diameter of 20µm and have good flow characteristics. Suitably the microspheres have a mean diameter of at least 2µm, preferably from 2 to 15µm, more preferably from 2 to 10µm, or most preferably from from 4 to 10µm. In a particular embodiment, the microspheres have a mean diameter of about 6µm. The maximum diameter of the particles is suitably 10µm, preferably 7µm, and preferably is 6µm.

The ratio of the first and second amounts of the agent used in the composition may vary depending upon the particular agents being employed, but in general, they will be in a ratio of from 1:2 to 2:1 and preferably at about 1:1.

These compositions are effective in the administration of pharmaceuticals generally but in particular, for the administration of a biologically active agent which is capable of generating a protective immune response in an animal, particularly a mammal, to which it is administered. Examples of such agents include antigenic polypeptides as well as nucleic acid sequences which may encode these polypeptides and which are known as "DNA" vaccines.

Suitable polypeptides are sub-unit vaccines and others, such as diptheria toxoid, tetanus toxoid, Botulinun toxin FHc and *Bacillus anthracis* protective antigen (PA).

As used herein the expression "polypeptide" encompasses proteins or epitopic fragments thereof.

Suitable polypeptides are sub-unit vaccines.

In a preferred embodiment, the composition of the invention comprises a biologically active agent which is capable of generating a protective immune response against *Yersinia pestis.* The agent is suitably a sub-unit vaccine, for example as described in WO 96/28551. The vaccine described and claimed there comprises a combination of the V antigen of *Y. pestis* or an immunologically active fragment thereof or a variant of these, and the F1 antigen of *Y. pestis* or an immunologically active fragment thereof or a variant of these.

As used herein, the term "fragment" refers to a portion of the basic sequence which includes at least one antigenic determinant. These may be deletion mutants. One or more epitopic region of the sequence may be joined together.

The expression "variant" refers to sequences of nucleic acids which differ from the base sequence from which they are derived in that one or more amino acids within the sequence are substituted for other amino acids. Amino acid substitutions may be regarded as "conservative" where an amino acid is replaced with a different amino acid with broadly similar properties. Non-conservative substitutions are where amino acids are replaced with amino acids of a different type. Broadly speaking, fewer non-conservative substitutions will be possible without altering the biological activity of the polypeptide. Suitably variants will be at least 60% homologous, preferably at least 75% homologous, and more preferably at least 90% homologous to the base sequence.

In particular, the biologically active agent comprises a combination of the V antigen of *Y. pestis,* and the F1 antigen of *Y. pestis* or an immunologically active fragment thereof. Suitably, both antigens are present in the first and second amounts within the composition, preferably in equal proportions.

It has been found that in a particularly preferred embodiment, each administration of microsphere preparation to a mouse contains from 30-50µg and most preferably about 40µg of each of said antigens. Preferably the dosage to humans and mammals would be of the same order in terms of mg/Kg.

Although the vaccine composition of the invention may further comprise an adjuvant in order to enhance the immune response to the biologically active material administered, this has not been found to be necessary and so preferably such adjuvants are omitted from the compositions. Where used, suitable adjuvants include pharmaceutically acceptable adjuvants such as Freund's incomplete adjuvant, alhydrogel,aluminium compounds and, preferably adjuvants which are known to up-regulate mucosal responses such as CTB, the non-toxic pentameric B subunit of cholera toxin (CT) or mutant heat-labile toxin (mLT) of *E.coli.*

Other adjuvant types are described in International Patent Application Nos. WO00/56282, WO00/56362 and WO00/56361.

Compositions of the invention are particularly suitable for intranasal application. They may comprise microspheres per se which are optionally preserved, for example by lyophilisation, or the microspheres may be combined with a pharmaceutically acceptable carrier or excipient. Examples of suitable carriers include solid or liquid carriers as is understood in the art. In a particularly preferred embodiment of the invention, the composition comprises a suspension of the microspheres in a liquid carrier and in particular phosphate-buffered saline.

The invention further provides a method of producing a pharmaceutical composition, which method comprises encapsulating pharmaceutically active agent in a polymeric material which has a high molecular weight, and in particular a molecular weight of 100KDa or more, so as to form microspheres with a maximum diameter of 20µm, and thereafter combining said microspheres with a further amount of said agent in a more highly bioavailable form.

The encapsulation is suitably achieved using a double emulsion solvent evaporation method, in which a first emulsion is formed with the pharmaceutically active agent, and the structural polymer, mixing this with an aqueous phase (suitably without structural polymer) to form a secondary emulsion, evaporating solvent and isolating small microspheres. In particular, the pharmaceutically active ingredient is dissolved or suspended in an aqueous solution which optionally includes an emulsifier such as PVA. The emulsifier, where present is suitably included at low concentrations for example of less than 5%w/v. This solution or suspension in then mixed with a solution of the high molecular weight structural polymer in an organic solvent such as dichloromethane. A primary emulsion is then formed, in particular by sonication of the mixture. The primary emulsion in then added to a secondary aqueous phase, which preferably includes an emulsifier with vigorous stirring. Solvent is then preferably evaporated, conveniently at room temperature. Microspheres can then be recovered, for example by centrifugation followed by lyophilisation.

In particular, the method of the invention is a method of producing a prophylactic or therapeutic vaccine, which method comprises encapsulating an agent which is capable of producing a protective immune response in a polymeric material which has a high molecular weight, and in particular a molecular weight of 100KDa or more, so as to form microspheres with a maximum diameter of less than 20µm, and thereafter combining said microspheres with a further amount of said agent in a more highly bioavailable form.

Methods of forming microspheres are well known in the art. They include emulsion techniques and spraydrying techniques.

Microspheres of the invention are suitably prepared using a double emulsion solvent evaporation method. Briefly, the biologically active agent, suitably in a lyophilised state, is suspended in an aqueous solution of a polymer such as polyvinyl alcohol (PVA) and methylcellulose. A solution of the high molecular weight polymer in an organic solvent such as dichloromethane, is added with vigorous mixing. The resultant emulsion is then dropped into a secondary aqueous phase, also containing the polymer (PVA or the like) with vigorous stirring. After addition, the organic solvent is allowed to evaporate off and the resultant microspheres separated.

Suitably the compositions are in unit dosage form. This will vary depending upon the nature of the active agent being employed, the nature of the patient, the condition being treated and other clinical factors. In general however, the composition of the invention will comprise approximately 2 to 10 wt% of active ingredient. The amount of high molecular weight polymer in the composition will be of the order of 70 to 98wt %.

In use, a reasonable dosage for nasal administration would be of the order of from 0.05g to 0.2g.

Compositions of the inventions include vaccine compositions, and in single dose vaccine compositions.

In a further aspect, the invention provides the use of the composition according to claim 1 in the preparation of a vaccine for the production of a protective immune response in a mammal.

The applicants have demonstrated that it is possible to protect experimental animals from inhalation challenge with *Y. pestis* using a single i.n. dose of a combined sub-unit vaccine in the form of a composition of the invention. The high molecular weight polymer utilised in the compositions of the invention appears to be particularly well suited to intra-nasal delivery.

The invention will now be particularly described by way of example.

### Example 1

### Preparation of Vaccine Composition

The V antigen of *Yersinia pestis* was expressed as a fusion protein with glutathione-s-transferase (GST) in *Escherischia coli*. The V protein was cleaved from the fusion with factor Xa (Boehringer Mannheim UK Ltd) for 18 hours at 22°C and recombinant V protein was purified by affinity adsorption, as described by S. Leary et al. (Infect. Immun. 1995, 255, 193-198). The F1 antigen was precipitated from the supernatant of *y. pestis* grown at 37°C in a chemically defined medium (pH 7.4), by the addition of 40% (w/v) ammonium sulphate and purified by repeated resuspension and centrifugation of the pellet in 20mM Tris-HC1 at pH8.

### Example 2

### Microencapsulation of subunits

Poly-L-lactide of molecular weight 100KDa (Polysciences Inc. USA) was used in a modification of the double emulsion solvent evaporation method (Y. Ogawa et al., Chem. Pharm. Bull., 36 (1988) 1095-1103). Briefly, 4.2mg of V and 5.9mg of lyophilised F1 antigen was suspended in 0.5ml of an aqueous 2.5% solution of polyvinyl alcohol (PVA) 13-23KDa (88% hydrolysed). This suspension was mixed with 250mg of poly-L-lactide of molecular weight 100KDa dissolved in 5ml of HPLC grade dichloromethane (DCM) and the mixture sonicated for 2 minutes at 60W on ice. The resultant primary emulsion was added to a secondary aqueous phase (75ml) containing 5%w/v PVA 13-23KDa (88% hydrolysed) which was vigorously stirred using a Silverson homogeniser (Silverson, UK) at maximum speed for 8 minutes. The solvent was then allowed to evaporate at ambient temperature, and the resultant microspheres (MDVF)were recovered by ultra-centrifugation and then lyophilised.

Lyophilised microspheres were characterised using standard procedures.

In this way, the F1 and V proteins were co-encapsulated into each sphere, such that each milligram of spheres contained 18-20µg of each protein (maximum 40µg of protein per milligram of microspheres).

### Example 3

### Characterisation of Microspheres

Microsphere morphology was investigated using a scanning electron microscope (Cambridge Instruments UK, Stereoscan 90). Particle size was determined by laser diffraction (Malvern Instruments Ltd., Shropshire UK). Formulated particles were found to have a volume mean diameter of 6µm. Microspheres were smooth and spherical when visualised using the scanning electron microscope.

### Example 4

### Single dose intra-nasal delivery of microencapsulated F1+V protects mice against challenge with Y.pestis

Free F1 and V proteins were added to a suspension of MDVF microspheres from Example 2 in sterile saline. Dosage units were prepared comprising spheres containing 18-20µg of each protein (maximum 40µg of protein) and 20µg of each of the free proteins.

Thus the total dose of F1 and V delivered was 40 µg per animal.

Mice were dosed nasally on one occasion only with a suspension of these microspheres, delivered in a 50µl volume of phosphate-buffered saline.

Control animals received 'empty' spheres which had been made in the same way as above but without encapsulating protein and which had been admixed with sufficient free F1 and V proteins to give a total dose per mouse of 40µg F1+ 40µg V.

Immunised mice were challenged sub-cutaneously at day 60 after the single priming dose with 10⁴ cfu of virulent *Y.pestis* and at day 74 with 10⁶ cfu *Y.pestis*. Survival of the immunised mice is shown in the table below.

**Table**

| Protection from parenteral challenge with Y.pestis GB following a single nasal dose of encapsulated F1+V, with admixed free protein. | | |
|---|---|---|
| Immunisation | Survival at 10⁴ cfu Y.pestis s.c. | Survival at 10⁶ cfu Y.pestis s.c. |
| MDVF +free F1+free V | 10/10 | 9/10 |
| Empty spheres+ 40µg free F1+40µg free V | 4/10 | 3/10 |

It appeared that the formulation of the invention had the effect of delivering an initial 'burst' of F1+V immunogens, followed by a more gradual controlled release of the encapsulated protein which was effective in ensuring such good survival rates.

## Claims

1. A pharmaceutical composition for administration to mucosal surfaces, which composition comprises an agent which is capable of generating a biological effect, a first amount of said agent being encapsulated within microspheres which comprise a polymer which has a molecular weight in excess of 94kDa and a maximum diameter of 20µm, and a second amount of said agent being in a form which has a higher bioavailability than said first amount.

2. A composition according to claim 1 wherein the agent which is capable of generating a biological effect is an agent which is capable of generating a protective immune response in an animal to which it is administered.

3. A composition according to claim 1 or claim 2 wherein said second amount of said agent is free in the composition or is at least partially adsorbed onto the surface of said microspheres.

4. A composition according to claim 1 or claim 2 wherein said second amount of said agent is encapsulated in a readily dispersible microsphere or vesicle.

5. A composition according to any one of the preceding claims wherein the polymer has a molecular weight of 100KDa or more.

6. A composition according to any one of the preceding claims wherein the polymer comprises poly-(L-lactide).

7. A composition according to any one of the preceding claims wherein the microspheres have a volume mean diameter of from 2-15µm.

8. A composition according to claim 7 wherein the microspheres have a volume mean diameter of from 2-10µm.

9. A composition according to any one of the preceding claims wherein the microspheres have a maximum diameter of 10µm.

10. A composition according to any one of the preceding claims wherein the biologically active agent is capable of generating a protective immune response against *Yersinia pestis*.

11. A composition according to claim 10 wherein the biologically active agent comprises a combination of the V antigen of *Y. pestis* or an immunologically active fragment thereof, and the F1 antigen of *Y. pestis* or an immunologically active fragment thereof.

12. A composition according to any one of the preceding claims which is in unit dosage form.

13. A composition according to claim 9 wherein the unit dosage form comprises a single shot vaccine.

14. A composition according to any one of the preceding claims which is adapted for intranasal application.

15. A composition according to claim 14 which comprises a suspension of microspheres in phosphate-buffered saline.

16. A method of producing a pharmaceutical composition, which method comprises encapsulating a pharmaceutically active agent in a polymeric material which has a high molecular weight, so as to form microspheres with a mean diameter of less than 20µm, and thereafter combining said microspheres with a further amount of said agent in a more highly bioavailable form.

17. A method according to claim 16 which is a method of producing a prophylactic or therapeutic vaccine, and wherein the pharmaceutically active agent is capable of producing a protective immune response.

18. A method according to claim 16 or claim 17 wherein the encapsulation is effected using a double emulsion solvent evaporation method, in which a first emulsion is formed with the pharmaceutically active agent, and the structural polymer, mixing this with an aqueous phase (suitably without structural polymer) to form a secondary emulsion, evaporating solvent and isolating small microspheres.

19. A pharmaceutical composition according to claim 1 for use in the production of a protective immune response in a mammal by administration to a mucosal surface of said mammal.

20. The use of a composition according to claim 1 in the preparation of a vaccine for the production of a protective immune response in a mammal.

## Patentansprüche

1. Pharmazeutische Zusammensetzung zur Anwendung an Schleimhautoberflächen, wobei die Zusammensetzung ein Mittel umfasst, das eine biologische Wirkung erzeugen kann, wobei eine erste Menge des Mittels in Mikrokügelchen eingekapselt ist, die ein Polymer, das ein Molekulargewicht von mehr als 94 kDa aufweist, und einen maximalen Durchmesser von 20 µm umfassen, und eine zweite Menge des Mittels in einer Form vorliegt, die eine höhere Bioverfügbarkeit als die erste Menge aufweist.

2. Zusammensetzung nach Anspruch 1, in der das Mittel, das eine biologische Wirkung erzeugen kann, ein Mittel ist, das eine schützende Immunantwort in einem Lebewesen erzeugen kann, dem es verabreicht wird.

3. Zusammensetzung nach Anspruch 1 oder Anspruch 2, in der die zweite Menge des Mittels frei in der Zusammensetzung vorliegt oder zumindest teilweise auf der Oberfläche der Mikrokügelchen adsorbiert ist.

4. Zusammensetzung nach Anspruch 1 oder Anspruch 2, in der die zweite Menge des Mittels in einem leicht dispergierbaren Mikrokügelchen oder Vesikel eingekapselt ist.

5. Zusammensetzung nach irgendeinem der vorangehenden Ansprüche, in der das Polymer ein Molekulargewicht von 100 kDa oder mehr aufweist.

6. Zusammensetzung nach irgendeinem der vorangehenden Ansprüche, in der das Polymer Poly(L-lactid) umfasst.

7. Zusammensetzung nach irgendeinem der vorangehenden Ansprüche, in der die Mikrokügelchen ein Volumenmittel des Durchmessers von 2-15 µm aufweisen.

8. Zusammensetzung nach Anspruch 7, in der die Mikrokügelchen ein Volumenmittel des Durchmessers von 2-10 µm aufweisen.

9. Zusammensetzung nach irgendeinem der vorangehenden Ansprüche, in der die Mikrokügelchen einen maximalen Durchmesser von 10 µm aufweisen.

10. Zusammensetzung nach irgendeinem der vorangehenden Ansprüche, in der das biologisch aktive Mittel eine schützende Immunantwort gegen *Yersinia pestis* erzeugen kann.

11. Zusammensetzung nach Anspruch 10, in der das biologisch aktive Mittel eine Kombination des V-Antigens von *Y. pestis* oder eines immunologisch aktiven Fragments desselben und des F1-Antigens von *Y. pestis* oder eines immunologisch aktiven Fragments desselben umfasst.

12. Zusammensetzung nach irgendeinem der vorangehenden Ansprüche, die in Dosierungseinheitsform vorliegt.

13. Zusammensetzung nach Anspruch 9, in der die Dosierungseinheitsform einen Einmalverabreichungs-Impfstoff umfasst.

14. Zusammensetzung nach irgendeinem der vorangehenden Ansprüche, die für die intranasale Verabreichung angepasst ist.

15. Zusammensetzung nach Anspruch 14, die eine Suspension von Mikrokügelchen in Phosphat-gepufferter Kochsalzlösung umfasst.

16. Verfahren zur Herstellung einer pharmazeutischen Zusammensetzung, wobei das Verfahren das Einkapseln eines pharmazeutisch aktiven Mittels in ein polymeres Material, das ein hohes Molekulargewicht aufweist, um Mikrokügelchen mit einem mittleren Durchmesser von weniger als 20 µm zu bilden, und danach das Vereinigen der Mikrokügelchen mit einer weiteren Menge des Mittels in einer stärker bioverfügbaren Form umfasst.

17. Verfahren nach Anspruch 16, das ein Verfahren zur Herstellung eines prophylaktischen oder therapeutischen Impfstoffs ist und in dem das pharmazeutisch aktive Mittel eine schützende Immunantwort erzeugen kann.

18. Verfahren nach Anspruch 16 oder Anspruch 17, in dem die Einkapselung unter Verwendung eines Doppelemulsions-Lösungsmittelverdampfungs-Verfahrens bewirkt wird, in dem eine erste Emulsion mit dem pharmazeutisch aktiven Mittel und dem Strukturpolymer gebildet wird, diese mit einer wässrigen Phase (geeignet ohne Strukturpolymer) gemischt wird, um eine zweite Emulsion zu bilden, das Lösungsmittel verdampft wird und kleine Mikrokügelchen isoliert werden.

19. Pharmazeutische Zusammensetzung nach Anspruch 1 zur Verwendung bei der Erzeugung einer schützenden Immunantwort in einem Säuger durch Verabreichung an eine Schleimhautoberfläche des Säugers.

20. Verwendung einer Zusammensetzung nach Anspruch 1 bei der Herstellung eines Impfstoffs für die Erzeugung einer schützenden Immunantwort in einem Säuger.

## Revendications

1. Composition pharmaceutique pour administration à des surfaces de muqueuses, ladite composition comprenant un agent qui est capable de générer un effet biologique, une première quantité dudit agent étant encapsulée à l'intérieur de microsphères qui comprennent un polymère qui a un poids moléculaire qui dépasse 94 kDa et un diamètre maximum de 20 µm, et une seconde quantité dudit agent étant sous une forme qui a une plus forte biodisponibilité que ladite première quantité.

2. Composition selon la revendication 1 dans laquelle l'agent qui est capable de générer un effet biologique est un agent qui est capable de générer une réponse immunitaire protectrice chez l'animal auquel il est administré.

3. Composition selon la revendication 1 ou 2 dans laquelle ladite seconde quantité dudit agent est libre dans la composition ou est au moins partiellement adsorbée sur la surface desdites microsphères.

4. Composition selon la revendication 1 ou 2 dans laquelle ladite seconde quantité dudit agent est encapsulée dans une microsphère ou une vésicule qui peut se disperser rapidement.

5. Composition selon l'une quelconque des revendications précédentes dans laquelle le polymère a un poids moléculaire de 100 kDa ou plus.

6. Composition selon l'une quelconque des revendications précédentes dans laquelle le polymère comprend du poly-(L-lactide).

7. Composition selon l'une quelconque des revendications précédentes dans laquelle les microsphères ont un diamètre moyen volumique de 2 à 15 µm.

8. Composition selon la revendication 7 dans laquelle les microsphères ont un diamètre moyen volumique de 2 à 10 µm.

9. Composition selon l'une quelconque des revendications précédentes dans laquelle les microsphères ont un diamètre maximum de 10 µm.

10. Composition selon l'une quelconque des revendications précédentes dans laquelle l'agent biologiquement actif est capable de générer une réponse immunitaire protectrice contre Yersinia pestis.

11. Composition selon la revendication 10 dans laquelle l'agent biologiquement actif comprend une combinaison de l'antigène V de Y. pestis ou un fragment immunologiquement actif de ce dernier, et l'antigène F1 de Y. pestis ou un fragment immunologiquement actif de ce dernier.

12. Composition selon l'une quelconque des revendications précédentes qui est sous forme de dosage unitaire.

13. Composition selon la revendication 9 dans laquelle la forme de dosage unitaire comprend un vaccin à prise unique.

14. Composition selon l'une quelconque des revendications précédentes qui est adaptée à une application intranasale.

15. Composition selon la revendication 14 qui comprend une suspension de microsphères dans un sérum physiologique sous tampon phosphate.

16. Procédé de production pour une composition pharmaceutique, ledit procédé comprenant l'encapsulation d'un agent pharmaceutiquement actif dans un matériau polymère qui a un haut poids moléculaire afin de former des microsphères avec un diamètre moyen inférieur à 20 µm, et ensuite de combiner lesdites microsphères avec une autre quantité dudit agent sous une forme plus fortement biodisponible.

17. Procédé selon la revendication 16 qui est un procédé de production d'un vaccin prophylactique ou thérapeutique, et dans lequel l'agent pharmaceutiquement actif est capable de produire une réponse immunitaire protectrice.

18. Procédé selon les revendications 16 ou 17 dans lequel l'encapsulation est effectuée au moyen d'un procédé d'évaporation de solvant sur une double émulsion, dans lequel on forme une première émulsion avec l'agent pharmaceutiquement actif et le polymère structurel, on la mélange avec une phase aqueuse (de façon appropriée sans polymère structurel) pour former une émulsion secondaire, on évapore le solvant et on isole les petites microsphères.

19. Composition pharmaceutique selon la revendication 1 utilisée pour la production d'une réponse immunitaire protectrice chez un mammifère par administration sur une surface de muqueuse dudit mammifère.

20. Utilisation d'une composition selon la revendication 1 dans la préparation d'un vaccin pour la production d'une réponse immunitaire protectrice chez un mammifère.
